# EUROPEAN PATENT SPECIFICATION

(11) **EP 2 032 529 B1**
(45) Date of publication and mention of the grant of the patent: **17.10.2012**
(21) Application number: 07796442.7
(22) Date of filing: 26.06.2007
(51) Int. Cl.: C07C 257/14, C23C 16/34, C23C 16/40

(54) **METAL(IV) TETRA-AMIDINATE COMPOUNDS AND THEIR USE IN VAPOR DEPOSITION**
METALL(IV)-TETRAAMIDINATVERBINDUNGEN UND IHRE VERWENDUNG IN DER AUFDAMPFUNG
COMPOSÉS DE TÉTRA-AMIDINATE DE MÉTAL (IV) ET LEUR UTILISATION DANS LE DÉPÔT EN PHASE VAPEUR

(30) Priority: 28.06.2006 US 817209 P; 17.10.2006 US 581986
(43) Date of publication of application: 11.03.2009
(73) Proprietor: President and Fellows of Harvard College, Cambridge, MA 02138 (US)
(72) Inventor: GORDON, Roy, G., Cambridge, MA 02138 (US); LEHN, Jean-Sebastien, Airlington, Massachusetts 02476 (US); LI, Huazhi, Somerville, MA 02143 (US)
(74) Representative: Gambell, Derek
(86) International application number: PCT/US2007/014768
(87) International publication number: WO 2008/002546

(56) References cited:
- WO-A-91/08322
- WO-A-2004/046417
- FIX R ET AL: "CHEMICAL VAPOR DEPOSITION OF TITANIUM, ZIRCONIUM, AND HAFNIUM NITRIDE THIN FILMS" CHEMISTRY OF MATERIALS, AMERICAN CHEMICAL SOCIETY, WASHINGTON, US, vol. 3, no. 6, 1 November 1991 (1991-11-01), pages 1138-1148, XP000276044 ISSN: 0897-4756

## Description

### Background of the Invention

### 1. Field of the Invention

This invention relates to novel compounds containing metals in the +4 oxidation state bonded to four amidinate ligands. This invention also relates to the use of these compounds as precursors for vapor deposition of metal-containing layers.

### 2. Description of the Related Art

Electrically insulating materials with high dielectric constants ("high-k dielectrics") are now being used in the manufacture of computer memories (dynamic random access memories, or DRAMs). Aluminum oxide and tantalum oxide are currently in commercial use in DRAMs, and oxides, nitrides and silicates of hafnium and zirconium are being tested as alternatives for future use. These high-k materials may also be used as insulators in future transistors in microelectronic devices.

Electrically conductive nitrides of metals such as tantalum, tungsten, hafnium, zirconium, titanium, niobium and molybdenum have a variety of applications and potential applications, such as barriers against the diffusion of copper, and as electrodes for capacitors and transistors in microelectronic devices. These refractory metals also find use as adhesion-promoting layers for copper, and as electrodes or electrical interconnections.

Vapor deposition is a preferred method for making these materials. Vapor deposition is a generic term that comprises chemical vapor deposition (CVD) and atomic layer deposition (ALD). In a CVD process, one or more vapors are delivered to a surface on which solid material is deposited; the chemical reactions that convert the vapor to a solid are initiated by means such as heat, light or electrical excitation (plasma activation). In an ALD process, two or more vapors are delivered alternately to the surface on which reactions take place to deposit a solid product. ALD is capable of depositing these materials inside the very narrow structures in modern DRAMs. CVD generally provides higher deposition rates than ALD, but with less uniform deposition inside very narrow holes.

Successful precursors for vapor deposition must be volatile, thermally stable, and highly reactive. Identifying compounds that meet all of these challenging requirements is difficult. Fully satisfactory precursors for metals such as hafnium, zirconium, tantalum, niobium, tungsten, molybdenum, tin, tellurium and uranium are not known. Halides, such as ZrCl₄, HfCl₄, and TaCl₅, have difficulty nucleating on some substrate surfaces, and the byproduct hydrochloric acid prevents fully conformal deposition inside narrow holes. Alkoxides and dialkylamides have less than optimal thermal stabilities. Organometallic compounds may lack suitable reactivity, leaving carbon as an impurity in the films. Thus there is a need for more volatile, thermally stable, and highly reactive sources for these metals.

We are aware of International patent publication WO 2004046417-A2 (Harvard College et al.) which describes metal films which are deposited with uniform thickness and excellent step coverage. Copper metal films were deposited on heated substrates by the reaction of alternating doses of copper(I) NN'-diisopropylacetamidinate vapor and hydrogen gas. Cobalt metal films were deposited on heated substrates by the reaction of alternating doses of cobalt(II) bis(N,N'-diisopropylacetamidinate) vapor and hydrogen gas. Nitrides and oxides of these metals can be formed by replacing the hydrogen with ammonia or water vapor, respectively. The films have very uniform thickness and excellent step coverage in narrow holes. Suitable applications include electrical interconnects in microelectronics and magnetoresistant layers in magnetic information storage devices.

W09/08322 describes the chemical vapor deposition of transition metal nitrides, e.g. titanium nitride.

Fix R. et al., Chemistry of materials, 3, 1138-1148 (1991) describes the chemical vapor deposition of titanium, zirconium, and hafnium nitride thin films.

### Summary of the Invention

Essential and optional features of the present invention are set out in the accompanying main and sub-claims respectively. One aspect of the invention includes novel compounds containing metals in the +4 oxidation state bonded to four amidinate ligands. In preferred embodiments, these ligands comprise *N,N'*-dialkylamidinate ligands. Preferred metals include hafnium, zirconium, tantalum, niobium, tungsten, molybdenum, tin, tellurium and uranium.

The compound has the structural formula 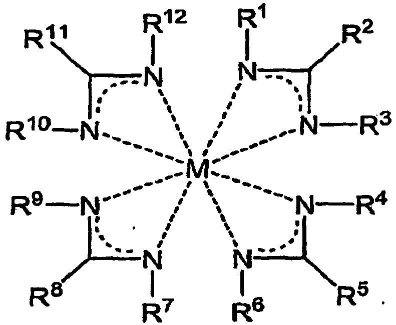 in which M is a metal in the +4 oxidation state and each of R¹ through R¹² are independently selected from the group consisting of hydrogen, hydrocarbon groups, substituted hydrocarbon groups, alkylsilyl, and alkylamino groups.

In one or more embodiments, the hydrocarbon group is selected from the group consisting of alkyl, cycloalkyl, alkenyl, cycloalkenyl, alkynyl and cycloalkynyl groups and the substituted hydrocarbon group consisting of fluoride derivatives of hydrocarbons.

In one or more embodiments, the metal M is selected from the group consisting of zirconium, hafnium, tin, tantalum, niobium, tungsten, molybdenum, uranium, rhenium, platinum, osmium, iridium, ruthenium, palladium, titanium, rhodium, vanadium, cerium and lead, or the metal M is selected from the group consisting of hafnium, zirconium, tantalum, niobium, tungsten, molybdenum, tin, tellurium and uranium.

In one or more embodiments, at least one of R¹ through R¹² is a lower alkyl having 5 or less carbons.

In one or more embodiments, R¹ through R¹² is selected from the group consisting of lower alkyls having 5 or less carbons and hydrogen.

In one or more embodiments, R¹, R³, R⁴, R⁶, R⁷, R⁹, R¹⁰ and R¹² are alkyl groups that are un-branched at the α-position.

Another aspect of the present invention includes a process for depositing films comprising metals using the novel compounds according to one or more embodiments of the invention. The process includes exposing a heated surface to the vapor of one or more volatile metal tetra-amidinate compounds. Exemplary deposition methods include Chemical Vapor Deposition (CVD) and Atomic Layer Deposition (ALD).

In one or more embodiments, the process includes exposing the substrate to a source of oxygen, and the thin film comprises a metal oxide.

In one or more embodiments, the source of oxygen comprises water vapor, or dioxygen, or ozone.

In one or more embodiments, the process includes exposing the substrate to a source of nitrogen, and the thin film comprises a metal nitride.

In one or more embodiments, the source of nitrogen comprises ammonia.

In one or more embodiments, the vapor is obtained by vaporizing a solid metal tetra-amidinate compound, or by vaporizing a liquid metal tetra-amidinate compound.

In one or more embodiments, the vapor is obtained by vaporizing a metal tetra-amidinate at a temperature in the range of 100 to 250°C.

In one or more embodiments, the surface is at a temperature in the range of about 200 to 500°C.

### Brief Description of the Drawings

The foregoing and various other aspects, features, and advantages of the present invention, as well as the invention itself, may be more fully appreciated with reference to the following detailed description of the invention when considered in connection with the following drawings. The drawings are presented for the purpose of illustration only and are not intended to be limiting of the invention, in which:
Fig. 1 is a schematic cross-sectional drawing of an ALD apparatus that can be used in some embodiments of the invention;
Fig. 2 is a drawing of the molecular structure of tetrakis(*N,N'*-di-isobutylacetamidinato) zirconium(IV); and
Fig. 3 is a drawing of the molecular structure of tetrakis(*N,N'-*dimethylpropionamidinato) hafnium(IV).
Fig. 4 is a graph of the evaporation rates of 4 hafnium amidinates.

### Detailed Description of the Invention

The present invention provides thermally stable, volatile metal compounds that are suitable for use in vapor deposition processes, including chemical vapor deposition and atomic layer deposition.

Preferred compounds include volatile metal(IV) tetrakis(*N,N'-*dialkylamidinates) complexes. Typically, these compounds are described by a monomeric formula 1, 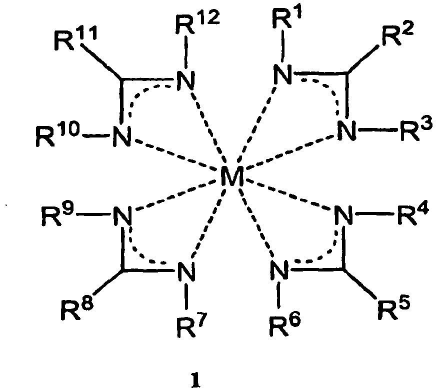 in which M is a metal in the +4 oxidation state and R¹ through R¹², e.g., Rⁿ, where n = 1-12, may be independently chosen from hydrogen, hydrocarbon groups such as alkyl, cycloalkyl, alkenyl, cycloalkenyl, alkynyl and cycloalkynyl groups and fluoride derivatives thereof, alkylsilyl or alkyl amino groups. Reference to Rⁿ applies equally to each of R¹ through R¹², unless otherwise specified.

In one or more embodiments, Rⁿ are lower alkyl groups containing 5 or less carbons. In one or more embodiments, Rⁿ are a mixture of hydrogen and lower alkyl groups. In preferred embodiments Rⁿ are chosen from the group comprising methyl, ethyl and n-propyl. These small alkyl groups are preferred because they are small enough to fit into the structure 1 with very stable chelate binding. In one or more embodiments, one or more of R¹, R³, R⁴, R⁶, R⁷, R⁹, R¹⁰, or R¹² are hydrocarbon groups lacking a branched α-carbon. As used herein, an α-carbon is a carbon bound directly to one nitrogen of an amidinate ligand. Alkyl groups that branch at the α-carbon, such as isopropyl, *sec*-butyl or *tert*-butyl, are less preferred because they are likely to cause too much crowding to fit into structure 1. Thus the branched alkyl groups will generally provide less stable metal amidinates. Nonetheless, branched groups are contemplated according to one or more embodiments, in particular, where a larger metal center is used or the branching occurs beyond the α-carbon.

Exemplary tetravalent metals that may be used in one or more embodiments of the invention include zirconium, hafnium, tin, tantalum, niobium, tungsten, molybdenum, uranium, rhenium, platinum, osmium, iridium, ruthenium, palladium, titanium, rhodium, vanadium, cerium, tellurium and lead. Tetravalent metal ions having relatively larger ionic radii form tetra-amidinate complexes that are particularly stable; those metals include zirconium, hafnium, tin, tantalum, niobium, tungsten, molybdenum, tellurium and uranium. Tetravalent metal ions having relatively smaller ionic radii that form tetra-amidinate include rhenium, platinum, osmium, iridium, ruthenium, palladium, titanium, rhodium and vanadium.

In one or more embodiments, the amidinate ligand is symmetric, e.g., the N-bound R groups such as R¹ and R³ or R⁴ and R⁶, etc., are the same in formula 1. In one or more embodiments, the amidinate ligand is asymmetric, e.g., R¹ and R³ are different in formula 1. In either embodiment, the carbon-bound R group, e.g., R² in formula 1, can be the same or different.

In one or more embodiments, metal tetrakis(*N,N*'-dialkylamidinate) compounds are prepared using *N,N'*-dialkylamidines. Symmetric amidines may be formed by condensation of amines with nitriles catalyzed by lanthanum trifluoromethanesulfonate (also known as lanthanum triflate), La(CF₃SO₃)₃:

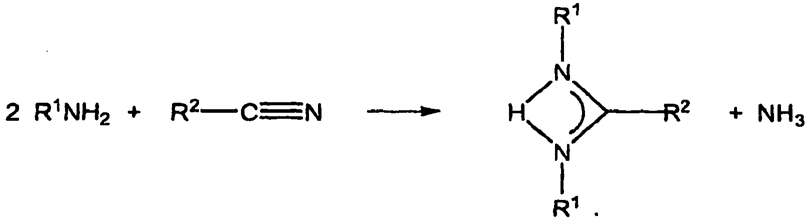

Amines in which R¹ is an alkyl group that is not branched at the α-position, such as *n*-propyl, *n*-butyl or isobutyl, react within a few hours to a few days at reflux temperature. Amines in which R¹ is a methyl or ethyl group are so volatile that they must be confined within a pressure vessel during the reaction. During the course of the reaction the byproduct ammonia may be released from the pressure vessel by a backpressure regulator. Complete reaction takes a few days at room temperature, or shorter periods at higher temperatures and pressures. A less expensive catalyst for this reaction can be made from mixed triflates of the naturally-occurring mixture of lanthanum metals ("misch metal").

Unsymmetrical amidines can be prepared according to the following reactions:

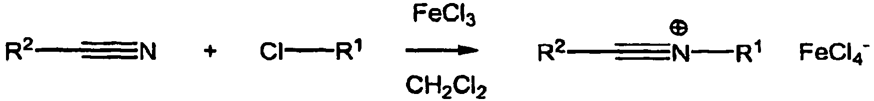

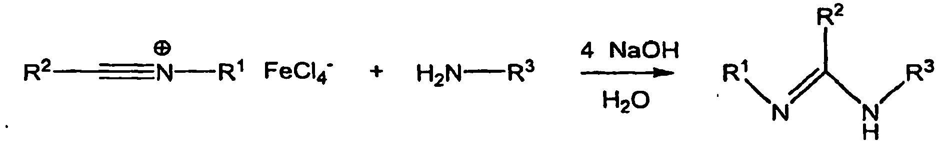

Metal amidinates can be prepared by exchange reactions in which a metal dialkylamide is reacted with an amidine. Alternately, the amidine can be converted to its alkali salt by reaction with butyllithium or with sodium amide or with potassium hydride. The alkali amidinate can then undergo a salt metathesis reaction with a metal chloride to form the metal amidinate. A more commonly-used method to form lithium amidinates is to react a carbodiimide with a lithium alkyl. This conventional synthetic route is more effective when the R¹ and R³ alkyl groups are branched at the α-position, because the corresponding carbodiimides are more stable.

The metal tetra-amidinate compounds may be used to form metal-containing films in a vapor deposition process. Vapors of the compounds according to one or more embodiments may be used to deposit materials such as metals, metal oxides, metal nitrides, metal oxynitrides, metal sulfides and the like. These vapor deposition processes include CVD and ALD. In CVD, a vapor of the metal tetra-amidinate is supplied to the surface, optionally along with a co-reactant gas or vapor. In ALD, a vapor of the metal tetra-amidinate and a co-reactant are supplied to the surface in alternating time periods. CVD processing is described, for example, in US Patent 5,139,999 and in the "Handbook of Chemical Vapor Deposition: Principles, Technology and Applications" by Hugh O. Pierson (2nd edition, Noyes Publications, 1999). ALD processing is described in US Patent 6,969,539 and in the article "Atomic Layer Deposition" by M. Ritala and M. Leskela, vol. 1, p. 103 of the Handbook of Thin Film Materials (Ed. H. Nalwa, Academic Press, 2002). Oxides may be formed using co-reactants such as water vapor, dioxygen, ozone, hydrogen peroxide and alcohols or a plasma formed from an oxygen-containing gas or vapor. Nitrides may be formed using co-reactants such as ammonia, a hydrazine or a plasma formed from a nitrogen-containing gas or vapor. Sulfides may be formed using co-reactants such as hydrogen sulfide or a plasma formed from a sulfur-containing gas or vapor.

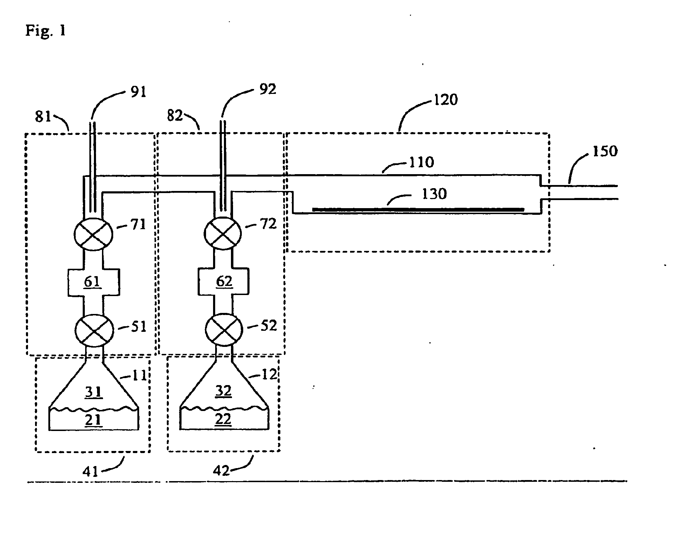

An apparatus for carrying out ALD is shown schematically in cross-section in Fig. 1. During operation of the ALD process, carrier gas, such as nitrogen, flows continuously from sources **91** and **92** through the deposition chamber **110** into pipe **150** to a trap and vacuum pump. A tetra-amidinate precursor **21** is held in vessel **11** that is heated in oven **41** to a temperature sufficient to form its vapor **31.** Vapor **31** of the tetravalent amidinate precursor flows into evacuated chamber **61** in oven **81** when valve **51** is opened. Valve **51** is then closed and valve **71** opened to allow an aliquot of precursor vapor to flow over the substrate **130** inside heated furnace **120.** Valve **71** is then closed and time is allowed for excess unreacted precursor to be purged from chamber **110** along with volatile reaction byproducts. The second reagent **22,** such as water or ammonia, is placed in vessel **12,** usually kept at room temperature. Vapor **32** of the second reagent is allowed to flow into vapor space **62** by opening valve **52,** which is then closed. Valve **72** is opened to allow an aliquot of the second reagent to flow into the deposition chamber **110.** Valve **72** is then closed and time is allowed for unreacted excess of the second reagent to be purged from the deposition chamber **110** along with volatile reaction byproducts. This cycle of operations is then repeated to build up the desired thickness of coating on substrate **130.**

An apparatus for carrying out CVD includes many similar features. The apparatus may include a vessel housing a tetra-amidinate precursor that is heated to a temperature to form its vapor. The tetra-amidinate precursor vapor flows from the vessel and into a heated furnace housing the substrate. Additional co-reactant vapors may be introduced into the heated furnace with the tetra-amidinate precursor, or the co-reactant vapors may be premixed with the tetra-amidinate vapor prior to their exposure to the heated substrate. An exhaust system removes by-products and unreacted reactants from the furnace.

The tetra-amidinate precursor may be used as a neat liquid, in solution in the appropriate solvent, or as a solid. Suitable deposition conditions, such as temperatures for vaporization and deposition, pressures, flow rates, and co-reactants may be readily determined by one of skill in the art. Exemplary ALD and CVD conditions include substrate temperatures of 200 to 500°C, and more preferably 300 to 400°C, vapor pressures in the range of 13.3 Pa to 1333.2 Pa (0.1 to 10 Torr), and more preferably 133.3 to 666.6 Pa (1 to 5 Torr), vaporization temperatures of about 100 to 250°C, and more preferably 150 to 200°C, ALD doses of 1 to 100 nmol cm⁻² of deposited surface, and more preferably 2 to 20 nmol cm⁻², and ALD exposures of 1.33 Pa sec to 1333.2 Pa-sec (0.01 Torr-sec to 10 Torr-sec), and more preferably 13.3 Pa sec to 133.3 Pa sec (0.1 to 1 Torr-sec). The ALD exposures needed to cover features with high aspect ratios increase approximately as the square of the aspect ratio.

### Examples

### Example 1. Synthesis of N,N'-di-iso-butylacetamidine.

A solution of *iso*-butylamine (7.3 g, 0.1 mol), acetonitrile (4.1 g, 0.1 mol) and lanthanum triflate, La(CF₃SO₃)₃, (1.2 g, 0.002 mol) was refluxed for 30 hr under nitrogen atmosphere. The unreacted starting material and byproduct 2,4,6trimethyl-1,3,5-triazine were removed by fractional distillation at 40 °C at around 0.2 Torr. Then the colorless *N,N'*-di-*iso*-butylacetamidine was distilled at 95 °C and 0.06 torr. Further purification was done by a second distillation. Yield: 6.4 g (75% based on iso-butylamine). ¹H NMR (benzene-*d₆* + a small amount of CD₃OD, ppm): 3.1 (d, 4, N*CH*₂), 1.9 (m, 2, *CH*(CH₃)₂), 1.7 (s, 3, C*CH*₃), 1.0 (d, 12, CH(*CH*₃)₂).

### Example 2. Synthesis of tetrakis(N,N'-di-iso-butylacetamidinato)zirconium(IV), Zr(ⁱBu-AMD)₄.

0.8 g (3 mmol) of tetrakis(dimethylamido)zirconium(IV), Zr(NMe₂)₄, was dissolved in 10 mL of toluene and then cooled to -30 °C for 30 minutes. To this solution was added 2.3 g (13.5 mmol) *N,N'*-di-*iso*-butylacetamidine, *ⁱ*Bu-AMD, and the mixture was heated at 90 °C overnight ligand exchange reaction). After cooling to - 30°C, a colorless crystalline material precipitated and was filtered out. Yield: 1.85 g (80%). ¹H NMR (benzene-*d₆*, ppm): 3.10 (d, 16, *J*= 6.9 Hz, N*CH*₂), 1.89 (m, 8, C*H*(CH₃)₂), 1.71 (s, 12, CC*H*₃), 1.00 (d, 48, *J*= 6.6 Hz, d, CH(*CH*₃)₂). ¹³C NMR (benzene-*d₆*, ppm): 174 (*C*CH₃), 55.76 (NCH₂), 31.74 (*C*H(CH₃)₂), 21.134 (CH(*C*H₃)₂, 12.10 (C*C*H₃). Anal. Calcd. for C₄₀H₈₄N₈Zr: C 62.53, H 11.02, N 14.58. Found: C 62.76, H 11.25, N 14.50.

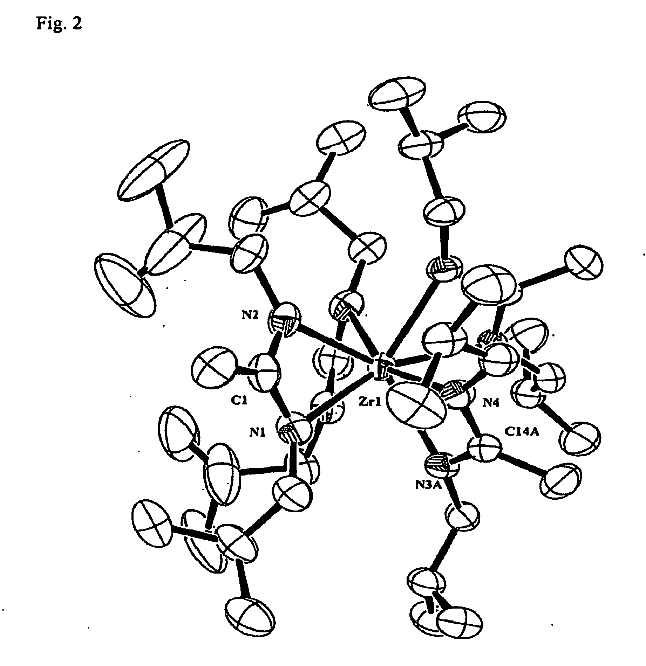

X-ray crystallography was used to determine the molecular structure of tetrakis(*N,N'*-di-iso-butylacetamidinato)zirconium(IV) shown in Figure 2, where the atoms are represented by 50% thermal ellipsoids and hydrogen atoms are omitted for clarity. Each molecule contains one zirconium atom surrounded by 8 nitrogen atoms from the four amidinate ligands.

The temperature at which half of the product evaporated during thermogravimetric analysis (TG), T_{1/2}, is 240°C, measured in flowing nitrogen gas at atmospheric pressure. This TG experiment also demonstrated that the compound has a high thermal stability and completely vaporizes with negligible residue.

### Example 3. Syntheses of tetrakis(N,N'-di-iso-butylacetamidinato)hafnium(IV), Hf(ⁱBu-AMD)₄.

This compound was prepared in a way similar to that described in Example 2 for Zr(*ⁱ*Bu-AMD)₄, starting from 3 mmol of tetrakis(dimethylamido)hafnium(IV), Hf(NMe₂)₄. The product was isolated as a white powder. Yield: 2.17g (85%). ¹H NMR (benzene-*d₆*, ppm): 3.15 (d, 16, *J*= 7.2 Hz, NC*H*₂), 1.87 (m, 8, *CH*(CH₃)₂), 1.70 (s, 12, CC*H*₃), 0.99 (d, 48, *J* = 6.8 Hz, CH(C*H*₃)₂). Anal. Calcd. for *C*₄₀H₈₄HfN₈: C 56.15, H 9.90, N 13.10. Found: 55.85, H 9.77, N 13.30.

The TG properties of this hafnium complex are similar to those of the zirconium complex described in Example 2.

### Example 4. Synthesis of N,N'-dimethylacetamidine and its lithium salt.

Anhydrous lanthanum triflate (3.00 g, 5.12 mmol) was placed into a pressure vessel. Dry acetonitrile (23.3 g, 0.568 mol) was condensed into the cold vessel. The vessel was cooled with liquid nitrogen and dry methylamine (53.1 g, 1.71 mol) was added. The vessel was sealed and allowed to warm to room temperature. Byproduct ammonia gas was released daily. Reaction was mostly complete after 3 days. Then the colorless *N,N'*-dimethylacetamidine was isolated by removing the byproduct *N-*methylacetamidine by sublimation at 20 °C and 5.33 Pa (0.04 torr). ¹H NMR (benzene-d₆, ppm): 2.60 (s, 6, N*CH*₃), 1.40 (s, 3, *CCH*₃).

The lithium salt of *N,N*'-dimethylacetamidine was prepared by dissolving one volume of *N,N'*-dimethylacetamidine in 5 volumes of dry ether and cooling the solution to -78°C. An equal molar amount of butyllithium dissolved in hexanes was added slowly while stirring. The reaction mixture was allowed to warm to room temperature. The ether and pentane were removed under reduced pressure. Then the white solid residue was dissolved in dry dioxane. The resulting solution of *N,N*'-dimethylacetamidinato-lithium in dioxane was used in some of the following examples.

### Example 5. Synthesis of tetrakis(N,N'-dimethylacetamidinato)zirconium(IV).

This compound was prepared by ligand exchange in a way similar to that described in Example 2 for Zr(*ⁱ*Bu-AMD)₄, using *N,N'*-dimethylacetamidine in place of *N,N*'-di-*iso*-butylacetamidine. Alternatively, ZrCl₄ was reacted with the lithium salt of *N,N'*-dimethylacetamidine dissolved in dioxane (salt metathesis reaction). This reaction mixture was heated to reflux for 8 hours. After evaporation of the dioxane, the solid residue was extracted with pentane. After decantation of the suspension to remove the precipitated lithium chloride, the pentane was evaporated under reduced pressure to yield the crude product, which was then purified by sublimation at 60°C and a pressure of 40 mbar. It can also be sublimed at atmospheric pressure at 160°C. The yield was 24% after sublimation, when the synthesis was done on a smal scale. ¹H NMR (benzene-d₆, ppm): 3.03 (s, 24, NC*H*₃), 1.57 (s, 12, CC*H*₃). ¹³C NMR (benzene-*d₆*, ppm): 175.99 (s, *C*CH₃), 34.55 (s, NCH₃), 9.84 (s, C*C*H₃). Anal. Calcd. for C₁₆H₃₆N₈Zr: C 44.51, H 8.40, N 25.95. Found: C 45.31, H 7.92, N 25.60 or, in a second analysis, C 43.30, H 8.76, N 24.87. This product is more volatile than tetrakis(*N,N'*-di-iso-butylacetamidinato)zirconium(IV), the product of Example 2 because its T_{1/2} value from the TG curve is 216°C with 0.6 % residue. Its melting point is about 168°C.

### Example 6. Synthesis of tetrakis(N,N'-dimethylacetamidinato)hafnium(IV).

This compound was prepared from HfCl₄ by the salt metathesis reaction described in Example 5. ¹H NMR (benzene-*d₆*, ppm): 3.07 (s, 24, NC*H*₃), 1.55 (s, 12, CC*H*₃). ¹³C NMR (benzene-*d₆*, ppm): 175.69 (s, *C*CH₃), 34.31 (s, N*C*H₃), 10.09 (s, C*C*H₃). Anal. Calcd. for C₁₆H₃₆HfN₈: C 37.03, H 6.99, N 21.59. Found: 37.00, H 6.89, N 21.34. This product is more volatile than tetrakis(*N,N'*-di-*iso*butylacetamidinato)hafnium(IV), the product of Example 3 because its T_{1/2} value from the TG curve is 221°C. Its residue after evaporation is negligible, less than 1%, and its melting point is about 171°C.

### Example 7. Synthesis of tetrakis(N,N'-dimethylpropionamidinato)zirconium(IV).

A dioxane solution of lithium *N,N'*-dimethylpropionamidinate was prepared by the method described in Example 4, using propionitrile in place of acetonitrile. This solution was then used with ZrCl₄ in the salt metathesis method described in Example 5 to prepare tetrakis(*N,N'*-dimethylpropionamidinato)zirconium(IV). This compound may also be prepared by a ligand exchange reaction similar to the one described in Example 2. ¹H NMR (benzene-*d₆*, ppm); 3.07 (s, 24, NC*H₃*), 2.10 (q, 8, *J* = 7.6 Hz, C*H₂*CH₃), 0.96 (t, 12, *J* = 7.6 Hz, CH₂C*H*₃). ¹³C NMR (benzene-*d₆*, ppm); 180.12 (s, *C*CH₂CH₃), 33.92 (s, N*C*H₃), 17.31 (s, CCH₂*C*H₃), 10.41 (s, C*C*H₂CH₃). Anal. Calcd. for C₂₀H₄₄N₈Zr: C 49.24, H 9.09, N 22.97. Found: 49.42, H 9.04, N 22.43. Its melting point is 109°C, which is low enough so that it is a liquid at a temperature high enough to vaporize it in a bubbler. Its T_{1/2} value from the TO curve is 245°C with a negligible residue of 0.6 %.

### Example 8. Synthesis of tetrakis(N,N'-dimethylpropionamidinato)hafnium(IV).

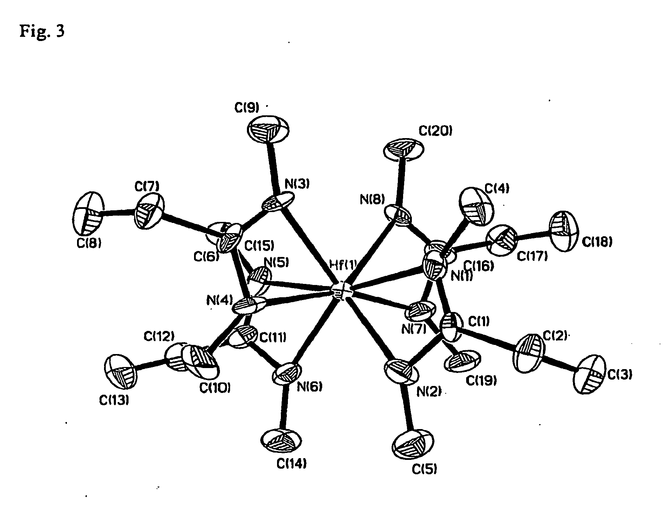

This compound was prepared from HfCl₄ by the salt metathesis reaction described in Example 7. It may also be prepared by a ligand exchange reaction similar to the one described in Example 1. ¹H NMR (benzene-*d₆*, ppm); 3.10 (s, 24, NC*H*₃), 2.08 (q, 8, *J* = 7.6 Hz, C*H*₂CH₃), 0.95 (t, 12, *J* = 7.6 Hz, CH₂C*H*₃). ¹³C NMR (benzene-*d₆*, ppm): 179.75 (s, *CC*H₂CH₃), 33.71 (s, N*C*H₃), 17.51 (s, CCH₂*C*H₃), 10.40 (s, C*C*H₂CH₃). Anal. Calcd. for C₂₀H₄₄HfN₈: C 41.77, H 7.71, N 19.48. Found: 42.32, H 8.11, N 19.18. Its melting point is 114°C, which is low enough so that it is a liquid at a temperature high enough to vaporize it in a bubbler. Its T_{1/2} value from the TG curve is 252°C, with a negligible non-volatile residue. X-ray crystallography was used to determine the molecular structure of tetrakis(N,N'- dimethylpropionamidinato)hafnium(IV) shown in Figure 3, where the atoms are represented by 50% thermal ellipsoids and hydrogen atoms are omitted for clarity. Each molecule contains one hafnium atom surrounded by 8 nitrogen atoms from the four amidinate ligands.

### Example 9. Synthesis of tetrakis(N,N'-dimethylbutyramidinato)zirconium(IV).

A dioxane solution of lithium N,N'-dimethylbutyramidinate was prepared by the method described in Example 4, using butyronitrile in place of acetonitrile. This solution was then used with ZrCl₄ in the salt metathesis method described in Example 5 to prepare tetrakis(*N,N'*-dimethylbutyramidinato)zirconium(IV). This compound may also be prepared by a ligand exchange reaction similar to the one described in Example 1. The compound is a liquid at room temperature, so it was purified by distillation instead of sublimation. ¹H NMR (benzene-*d₆*, ppm); 3.11 (s, 24, NC*H*₃), 2.15 (t, 8, *J*= 8.0 Hz, CC*H*₂CH₂CH₃), 1.49 (m, 8, CCH₂C*H*₂CH₃), 0.90 (t, 12, *J* = 6.8 Hz, CCH₂CH₂C*H*₃). ¹³C NMR (benzene-*d₆*, ppm). 179.27 (s, *C*CH₂CH₂CH₃), 34.28 (s, N*C*H₃), 26.14 (s, CCH₂*C*H₂CH₃), 19.82 (s, CCH₂CH₂*C*H₃), 14.47 (s, C*C*H₂CH₂CH₃). Anal. Calcd. for C₂₄H₅₂N₈Zr: C 52.99, H 9.63, N 20.60. Found: 53.63, H 9.87, N 20.89. Its T_{1/2} value is 246°C and it evaporates leaving a negligible residue.

### Example 10. Synthesis of tetrakis(N,N'-dimethylbutyramidinato)hafnium(IV).

This compound was prepared from HfCl₄ by the salt metathesis reaction described in Example 9. It may also be prepared by a ligand exchange reaction similar to the one described in Example 1. The compound is a liquid at room temperature, so it was purified by distillation instead of sublimation. ¹H NMR (benzene-*d₆*, ppm): 3.15 (s, 24, NC*H*₃), 2.13 (t, 8, *J*= 8.0 Hz, CC*H*₂CH₂CH₃), 1.49 (m, 8, CCH₂C*H*₂CH₃), 0.89 (t, 12, *J*= 6.8 Hz, CCH₂CH₂C*H*₃). ¹³C NMR (benzene-*d*₆, ppm); 178.87 (s, *C*CH₂CH₂CH₃), 34.08 (s, N*C*H₃), 26.29 (s, CCH₂*C*H₂CH₃), 19.82 (s, CCH₂CH₂*C*H₃), 14.41 (s, C*C*H₂CH₂CH₃). Anal. Calcd. for C₂₄H₅₂HfN₈: C 45.67, H 8.30, N 17.75. Found: 45.31, H 8.81, N 17.61. Its T_{1/2} value is 252°C and it evaporates leaving a negligible residue.

### Example 11. Synthesis of tetrakis(N,N'-diethlyacetamidinato)zirconium(IV)

A dioxane solution of lithium *N,N'*-diethylacetamidinate was prepared by the method described in Example 4, using ethylamine in place of methylamine. This solution was then used with ZrCl₄ in the salt metathesis method described in Example 5 to prepare tetrakis(*N,N*'-diethylacetamidinato)zirconium(IV). It may also be prepared by a ligand exchange reaction similar to the one described in Example 1. ¹H NMR (benzene-d₆, ppm); 3.32 (q, 16, *J=* 7.2 Hz, NC*H₂*CH₃), 1.63 (s, 12, CC*H*₃), 1.10 (t, 24, *J*= 7.2 Hz, NCH₂C*H*₃). ¹³C NMR (benzene-*d₆*, ppm); 173.59 (s, *C*CH₃), 41.38 (s, N*C*H₂CH₃), 18.00 (s, NCH₂*C*H₃), 10.20 (s, C*C*H₃). Anal. Calcd. for C₂₄H₅₂N₈Zr: C 52.99, H 9.63, N 20.60. Found: 52.86, H 9.40, N 20.99. Its T_{1/2} value is 242°C and it evaporates leaving a negligible residue.

### Example 12. Synthesis of tetrakis(N,N'-diethlyacetamidinato)hafnium(IV).

This compound was prepared from HfCl₄ by the salt metathesis method described in Example 11. It may also be prepared by a ligand exchange reaction similar to the one described in Example 1. ¹H NMR (benzene-*d₆*, ppm); 3.32 (q, 16, *J=* 7.2 Hz, NC*H*₂CH₃), 1.63 (s, 12, CC*H*₃), 1.10 (t, 24, *J* = 7.2 Hz, NCH₂C*H*₃. ¹³C NMR (benzene-*d₆*, ppm): 173.07 (s, *C*CH₃), 41.08 (s, N*C*H₂CH₃), 18.00 (s, NCH₂*C*H₃), 10.59 (s, C*C*H₃). Anal. Calcd. for C₂₄H₅₂N₈Hf: C 45.67, H 8.30, N 17.75. Found: 46.17, H 7.93, N 17.27. Its T_{1/2} value is 264°C and it evaporates leaving a negligible residue.

### Example 13. Synthesis of tetrakis(N,N'-diethylacetamidinato)tantalum(IV).

This compound was prepared in two steps from tantalum pentachloride. The first step involved the addition under nitrogen at -78°C of an ether solution of tantalum pentachloride (0.95 mmol, 331 mg in 20 mL ether) to a solution of lithium *N,N*'-diethylacetamidinate (2 mmol in 20 mL ether, prepared in situ from the amidine and a hexanes solution (2.6 M) of *n*-butyl lithium). The intermediate, tentatively described as trischloro-bis(*N,N'*-diethylacetamidinato)tantalum(V) was partially soluble in ether, giving an orange solution. Some dioxane was added to help dissolve the intermediate, and two more equivalents of lithium *N,N*'-diethylacetamidinate (2 mmol in 20 mL ether, prepared in situ from the amidine and a hexanes solution (2.6 M) of *n*-butyl lithium) were added at room temperature. One equivalent of sodium amalgam was also added (22.8 mg, in a mercury amalgam having 0.645 % sodium by weight, 3.53 g). The solution was stirred overnight at room temperature. The solution turned dark purple within 12 hours. The ether was stripped under vacuum, and pentane (20 mL) was added. The purple solution was decanted and separated from the mercury and the insoluble materials. It was dried under vacuum and yielded the product, a purple solid, which can tentatively be described as tetrakis(*N,N*'-diethytacetamidinato)tantalum(IV). The product could be sublimed under vacuum, and the sublimed fraction could also be sublimed again without decomposition at 150°C at a pressure of 133.3 Pa (0.01 mmHg). Although the product contained impurities; its color and the fact it could be sublimed under vacuum with no decomposition indicate it is likely to be a volatile tantalum(IV) amidinate.

### Example 14. Synthesis of other metal(IV) tetra-amidinates.

Compounds containing other metal centers can be prepared in ways similar to those described in Example 3, by using a suitable metal source in place of ZrCl₄. For example, tetrakis(*N,N*'-dimethylacetamidinato) tungsten(IV) is prepared using tungsten(IV) chloride, WCl₄; tetrakis(*N,N'*-dimethylacetamidinato)tin(IV) is prepared using tin(IV) chloride, SnCl₄; tetrakis(*N,N*'-dimethylacetamidinato)tellurium(IV) is prepared from TeCl₄; and **tetrakis(*N,N*'-dimethylacetamidinato)uranium(IV)** is prepared in using uranium(IV) chloride, UCl₄.

### Example 15. Atomic layer deposition of hafnium oxide from tetrakis(N,N'-dimethylbutyramidinato)hafnium(IV) and ozone.

10 nmol cm⁻² doses of the vapor of tetrakis(*N,N*'-dimethylpropionamidinato)hafnium(IV) from a direct liquid injection system at 200°C are introduced with an exposure of 1333.2Pa-sec (10 Torr-sec) in to an ALD reactor at 400°C, alternately with 20 nmol cm⁻² doses of ozone at an exposure of 1333.2Pa-sec (10 Torr-sec). A film of hafnium oxide is deposited conformally inside narrow holes with high aspect ratio of 80:1.

### Example 16. Atomic layer deposition of hafnium oxide from tetrakis(N,N'-dimethylbutyramidinato)hamium(IV) and water vapor.

10 nmol cm⁻² doses of the vapor of tetrakis(*N*,*N*'-dimethylpropionamidinato)hafnium(IV) from a direct liquid injection system at 200°C are introduced with an exposure of 10 Torr-sec in to an ALD reactor at 400°C, alternately with 20 nmol cm⁻² doses of water vapor at an exposure of 1333.2 Pa-sec (10 Torr-sec). A film of hafnium oxide is deposited conformally inside narrow holes with high aspect ratio of 80:1.

### Example 17. Atomic layer deposition of hafnium nitride from tetrakis(N,N'-dimethylbutyramidinato)hafnium(IV) and ammonia.

10 nmol cm⁻² doses of the vapor of tetrakis(*N,N*'-**dimethylpropionamidinato)hafnium(**IV) from a direct liquid injection system at 200°C are introduced with an exposure of 10 Torr-sec in to an ALD reactor at 400°C, alternately with 20 nmol cm⁻² doses of ammonia at an exposure of 10 Torr-sec. A film of hafnium nitride is deposited conformally inside narrow holes with high aspect ratio of 80:1.

### Example 18. Synthesis of N,N'-dimethylformamidine.

An excess of dry methylamine (39 g, 1.26 mol) was condensed onto a mixture of dry sodium cyanide (30.2 g, 0.617 mol), dry methylamine hydrochloride (41.6 g, 0.617 mol), and lanthanum triflate (5.2 g, 8.88 mmol) at -196 °C in a closed pressure vessel. The mixture was allowed to warm to room temperature and then sonicated for 72 hours at 40-50 °C. The volatile gases were allowed to escape from the reaction vessel at room temperature, and dry methylamine (10 g, 0.322 mol) was condensed onto the cooled reaction mixture. This cycle of evaporation, methylamine addition and sonication was repeated two more times. All volatiles were finally released by slowly venting the reaction vessel at room temperature. Diethyl ether (50 mL) was added, and the reaction mixture was poured into a large flask under nitrogen. All volatiles were transferred from the reaction mixture at room temperature into a large Schienk flask at -196 °C under vacuum through a bulb-to-bulb transfer of vapor. *N,N*'-Dimethylformamidine was obtained as a colorless solution in ether. ¹H NMR (benzene-*d₆* + a small amount of CD₃OD, ppm): 7.29 (s, 1, C*H*), 2.65 (br, 6, NC*H*₃).

### Example 19. Synthesis of N,N'-dimethylformamidine.

The potassium salt of methylamine is heated to about 100 °C until it decomposes to yield the same product *N,N*'-dimethylformamidine as Ex. 18.

### Example 20. Synthesis of tetrakis(N,N'-dimethylformamidinato)hafnium(IV).

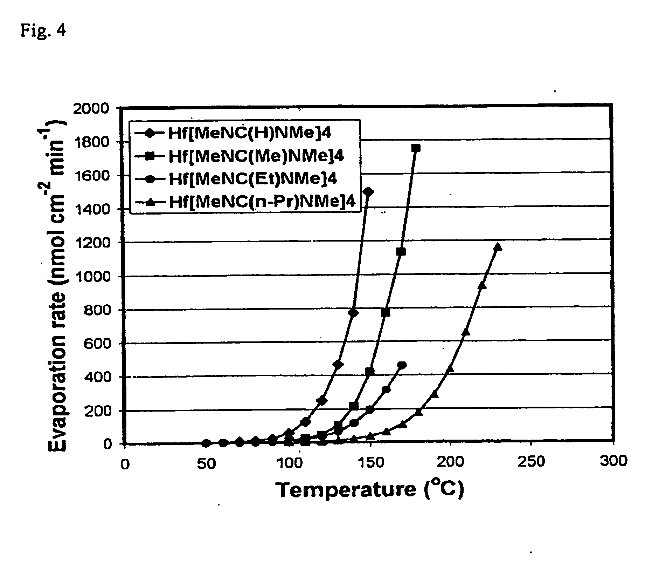

1.16 g (3.29 mmol) of tetrakis(dimethylamido)hafnium(IV), Hf(NMe₂)₄, was dissolved in 20 mL of diethyl ether and then cooled to -30 °C for 30 minutes. To this solution was added 0.968 g (13.4 mmol) *N,N'*-dimethylformamidine in 20 mL diethyl ether and the mixture was stirred at room temperature overnight. A small amount of white byproduct precipitated (probably due to monomethylformamidine impurity in the dimethylformamidine). The clear supernatant ether solution was decanted and evaporated under reduced pressure to yield the product as white crystals. Its melting point is 140-142 °C. ¹H NMR (benzene-*d₆*, ppm): 3.03 (s, 24, *NCH₃),* 8.00 (s, 4, *CH).* ¹³C NMR (benzene-*d₆*, ppm): 171.60 (CH), 38.67 (N*C*H₃). Its T_{1/2} value is 187 °C and it evaporates leaving a negligible residue.
The isothermal thermogravimetric analyses of evaporation rates are compared in Fig. 4 for 4 different hafnium amidinates (from Examples 6, 8, 10 and 19). These measurements show that at each temperature tetrakis(*N,N*'-dimethylformidinato)hafnium(IV) (Ex. 20) has the highest evaporation rate. At bubbler temperatures above 142 °C (its melting point) it is a liquid, so that its high evaporation rate is very reproducible. Thus in many applications, tetrakis(*N,N*'-dimethylformidinato)hafnium(IV) is a preferred precursor for vapor deposition of hafnium-containing materials. In applications for which a liquid at room temperature is required, it may be supplied as a liquid solution in a solvent with a similar boiling point. Suitable solvents include polyethers, such as triglyme or tetraglyme. Alternatively, the room-temperature liquid tetrakis(*N,N*'-dimethylbutyramidinato)hafnium(IV) may be used for neat liquid delivery to a vaporization system.

### Example 21. Synthesis of tetrakis(N,N'-dimethylformamidinato)zirconium(IV).

The steps of Ex. 20 are repeated with tetrakis(dimethylamido)zirconium(IV), Zr(NMe₂)₄, in place of tetrakis(dimethylamido)hafnium(IV), Hf(NMe₂)₄. Tetrakis(*N,N-*dimethylfommmidinato)zirconium(IV) is obtained, having similar properties to tetrakis(*N,N*'-dimethylformidinato)hafnium(IV).

## Claims

1. A compound having the structural formula 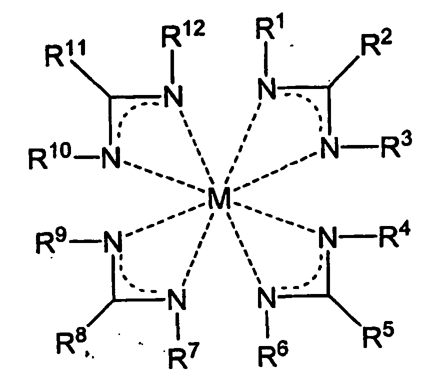 in which M is a metal in the +4 oxidation state and each of R¹ through R¹² are independently selected from the group consisting of hydrogen, hydrocarbon groups, substituted hydrocarbon groups, alkylsilyl, and alkyl amino groups.

2. The compound of claim 1,
a) wherein the hydrocarbon group is selected from the group consisting of alkyl, cycloalkyl, alkenyl, cycloalkenyl, alkynyl and cycloalkynyl groups, or
b) wherein the substituted hydrocarbon group is selected from the group consisting of fluoride derivatives of the hydrocarbon groups, or
c) wherein metal M is selected from the group consisting of zirconium, hafnium, tin, tantalum, niobium, tungsten, molybdenum, uranium, rhenium, platinum, osmium, iridium, ruthenium, palladium, titanium, rhodium, vanadium, cerium and lead, or
d) in which the metal M is selected from the group consisting of hafnium, zirconium, tantalum, niobium, tungsten, molybdenum, tin, tellurium and uranium, or
e) in which the metal M is of zirconium, or
f) in which the metal M is of hafnium, or
g) wherein at least one of R¹ through R¹² is lower alkyl having 5 or less carbons, or
h) wherein R¹ through R¹² is selected from the group consisting of lower alkyls having 5 or less carbons and hydrogen, or
i) wherein R¹, R³, R⁴, R⁶, R⁷, R⁹, R¹⁰and R¹² are alkyl groups that are un-branched at the α-position.

3. A process for forming a thin film comprising a metal, comprising:
exposing a heated surface to the vapor of one or more compounds of claims 1 or 2.

4. The process of claim 3,
a) wherein the hydrocarbon group is selected from the group consisting of alkyl, cycloalkyl, alkenyl, cycloalkenyl, alkynyl and cycloalkynyl groups, or
b) wherein the substituted hydrocarbon group is selected from the group consisting of fluoride derivatives of the hydrocarbon group.

5. The process of claim 3,
a) in which metal M is selected from the group consisting of zirconium, hafnium, tin, tantalum, niobium, tungsten, molybdenum, uranium, rhenium, platinum, osmium, iridium, ruthenium, palladium, titanium, rhodium, vanadium, cerium and lead, or
b) in which the metal M is selected from the group consisting of hafnium, zirconium, tantalum, niobium, tungsten, molybdenum, tin, tellurium and uranium, or
c) in which the metal M is of zirconium , or
d) in which the metal M is of hafnium, or
e) wherein at least one of R¹ through R¹² is a lower alkyl having 5 or less carbons, or
f) wherein R¹ through R¹² is selected from the group consisting of lower alkyls having 5 or less carbons and hydrogen.

6. The process of claim 3, in which the substrate is also exposed to a source of oxygen, and the thin film comprises a metal oxide.

7. The process of claim 6, in which the source of oxygen comprises water vapor.

8. The process of claim 6, in which the source of oxygen comprises dioxygen.

9. The process of claim 6, in which the source of oxygen comprises ozone.

10. The process of claim 3, in which the substrate is also exposed to a source of nitrogen, and the thin film comprises a metal nitride.

11. The process of claim 10, in which the source of nitrogen comprises ammonia.

12. The process of claim 3, wherein
a) the film is deposited in a CVD process, or
b) the film is deposited in a ALD process, or
c) the vapor is obtained by vaporizing a solid metal tetra-amidinate compound, or
d) the vapor is obtained by vaporizing a liquid metal tetra-amidinate compound or
e) the vapor is obtained by vaporizing a metal tetra-amidinate at a temperature in the range of 100 to 250°C, or
f) the surface is at a temperature in the range of 200 to 500°C.

## Patentansprüche

1. Verbindung mit der Strukturformel 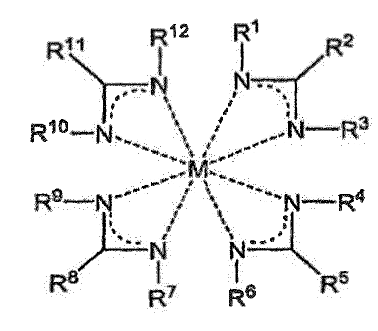 in welcher M ein Metall in dem +4 Oxidationszustand ist, und jedes von R¹ - R¹² unabhängig aus der aus Wasserstoff, Kohlenwasserstoffgruppen, substituierten Kohlenwasserstoffgruppen, Alkylsilyl und Alkylaminogruppen bestehenden Gruppe ausgewählt ist.

2. Verbindung nach Anspruch 1,
a) wobei die Kohlenwasserstoffgruppe aus der aus Alkyl, Cycloalkyl, Alkenyl, Cycloalkenyl, Alkynyl und Cycloalkynylgruppen bestehenden Gruppe ausgewählt ist, oder
b) wobei die substituierte Kohlenwasserstoffgruppe aus der aus Fluoridderivaten der Kohlenwasserstoffgruppe bestehenden Gruppe ausgewählt ist, oder
c) wobei das Metall M aus der aus Zirkonium, Hafnium, Zinn, Tantal, Niob, Wolfram, Molybdän, Uran, Rhenium, Platin, Osmium, Iridium, Ruthenium, Palladium, Titan, Rhodium, Vanadium, Cer und Blei bestehenden Gruppe ausgewählt ist, oder
d) in welcher das Metall aus der aus Hafnium, Zirkonium, Tantal, Niob, Wolfram, Molybdän, Zinn, Tellur und Uran bestehenden Gruppe ausgewählt ist, oder
e) in welcher das Metall M Zirkonium ist, oder
f) in welcher das Metall M Hafnium ist, oder
g) wobei wenigstens eines von R¹ - R¹² ein Niederalkyl mit 5 oder weniger Kohlenstoffatomen ist, oder
h) wobei R¹ - R¹² aus der aus Niederalkylen mit 5 oder weniger Kohlenstoffatomen und Wasserstoff bestehenden Gruppe ausgewählt ist, oder
i) wobei R¹, R³, R⁴, R⁶, R⁷, R⁹, R¹⁰ und R¹² Alkylgruppen sind, die an der α-Position unverzweigt sind.

3. Verfahren zum Erzeugen eines ein Metall aufweisenden dünnen Films mit den Schritten:
Aussetzen einer erhitzten Oberfläche dem Dampf von einer oder mehreren Verbindungen der Ansprüche 1 oder 2.

4. Verfahren nach Anspruch 3,
a) wobei die Kohlenwasserstoffgruppe aus der aus Alkyl, Cycloalkyl, Alkenyl, Cycloalkenyl, Alkynyl und Cycloalkynylgruppen bestehenden Gruppe ausgewählt wird, oder
b) wobei die substituierte Kohlenwasserstoffgruppe aus der aus Fluoridderivaten der Kohlenwasserstoffgruppe bestehenden Gruppe ausgewählt ist.

5. Verfahren nach Anspruch 3,
a) in welchem das Metall M aus der aus Zirkonium, Hafnium, Zinn, Tantal, Niob, Wolfram, Molybdän, Uran, Rhenium, Platin, Osmium, Iridium, Ruthenium, Palladium, Titan, Rhodium, Vanadium, Cer und Blei bestehenden Gruppe ausgewählt ist, oder
b) in welchem das Metall aus der aus Hafnium, Zirkonium, Tantal, Niob, Wolfram, Molybdän, Zinn, Tellur und Uran bestehenden Gruppe ausgewählt ist, oder
c) in welchen das Metall M Zirkonium ist, oder
d) in welchem das Metall M Hafnium ist, oder
e) wobei wenigstens eines von R1 - R12 ein Niederalkyl mit 5 oder weniger Kohlenstoffatomen ist, oder
f) wobei R1 - R12 aus der aus Niederalkylen mit 5 oder weniger Kohlenstoffatomen und Wasserstoff bestehenden Gruppe ausgewählt ist, oder

6. Verfahren nach Anspruch 3, in welchem das Substrat auch einer Sauerstoffquelle ausgesetzt wird, und der dünne Film ein Metalloxid aufweist.

7. Verfahren nach Anspruch 6, in welchem die Sauerstoffquelle Wasserdampf aufweist.

8. Verfahren nach Anspruch 6, in welchem die Sauerstoffquelle Disauerstoff aufweist.

9. Verfahren nach Anspruch 6, in welchem die Sauerstoffquelle Ozon aufweist.

10. Verfahren nach Anspruch 3, in welchem das Substrat auch einer Stickstoffquelle ausgesetzt wird und der dünne Film ein Metallnitrid aufweist.

11. Verfahren nach Anspruch 10, in welchem die Stickstoffquelle Ammoniak aufweist.

12. Verfahren nach Anspruch 3, wobei
a) der Film in einem CVD-Verfahren abgeschieden wird, oder
b) der Film in einem ALD-Verfahren abgeschieden wird, oder
c) der Dampf durch Verdampfen einer festen Metall-Tetra-Amidinatverbindung erhalten wird, oder
d) der Dampf durch Verdampfen einer flüssigen Metall-Tetra-Amidinatverbindung erhalten wird, oder
e) der Dampf durch Verdampfen eines Metall-Tetra-Aminidats bei einer Temperatur in einem Bereich von 100 bis 250 °C erhalten wird, oder
f) sich die Oberfläche auf einer Temperatur in dem Bereich von 200 bis 500 °C befindet.

## Revendications

1. Composé ayant la formule structurelle : 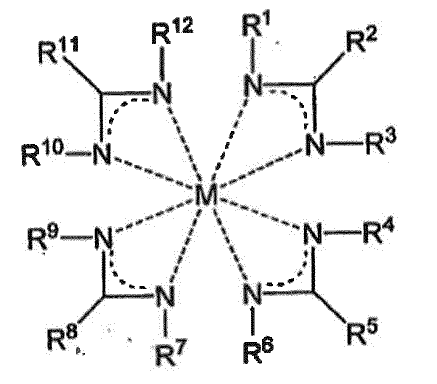 dans laquelle M est un métal dans l'état d'oxydation +4 et chacun de R¹ à R¹² est sélectionné de manière indépendante parmi le groupe constitué de l''hydrogène, de groupes hydrocarbonés, de groupes hydrocarbonés substitués, d'alkylsilyle, et de groupes amino alkyle.

2. Composé selon la revendication 1,
a) dans lequel le groupe hydrocarboné est sélectionné parmi le groupe constitué d'alkyle, cycloalkyle, alkényle, cycloalkényle, de groupes alkynyle et cycloalkynyle, ou
b) dans lequel le groupe hydrocarboné substitué est sélectionné parmi le groupe constitué de dérivés de fluorure des groupes hydrocarbonés, ou
c) dans lequel le métal M est sélectionné parmi le groupe constitué de zirconium, hafnium, étain, tantale, niobium, tungstène, molybdène, uranium, rhénium, platine, osmium, iridium, ruthénium, palladium, titane, rhodium, vanadium, cérium et plomb, ou
d) dans lequel le métal M est sélectionné parmi le groupe constitué de hafnium, zirconium, tantale, niobium, tungstène, molybdène, étain, tellure et uranium, ou
e) dans lequel le métal M est zirconium, ou
f) dans lequel le métal M est hafnium, ou
g) dans lequel au moins un parmi R¹ à R¹² est un alkyle inférieur ayant cinq carbones ou moins, ou
h) dans lequel R¹ à R¹² est sélectionné parmi le groupe constitué d'allyles inférieurs ayant cinq carbones ou moins et l'hydrogène, ou
i) dans lequel R¹, R³, R⁴, R⁶, R⁷, R⁹, R¹⁰ et R¹² sont des groupes alkyle qui sont non ramifiés dans la position α.

3. Procédé pour former un film mince comprenant un métal, comprenant :
l'exposition d'une surface chauffée à la vapeur d'un ou plusieurs composés selon les revendications 1 ou 2.

4. Procédé selon la revendication 3,
a) dans lequel le groupe hydrocarboné est sélectionné parmi le groupe constitué d'alkyle, cycloalkyle, alkényle, cycloalkényle, de groupes alkynyle et cycloalkynyle, ou
b) dans lequel le groupe hydrocarboné substitué est sélectionné parmi le groupe constitué de dérivés de fluorure des groupe hydrocarbonés.

5. Procédé selon la revendication 3,
a) dans lequel le métal M est sélectionné parmi le groupe constitué de zirconium, hafnium, étain, tantale, niobium, tungstène, molybdène, uranium, rhénium, platine, osmium, iridium, ruthénium, palladium, titane, rhodium, vanadium, cérium et plomb, ou
b) dans lequel le métal M est sélectionné parmi le groupe constitué de hafnium, zirconium, tantale, niobium, tungstène, molybdène, étain, tellure et uranium, ou
c) dans lequel le métal M est zirconium, ou
d) dans lequel le métal M est hafnium, ou
e) dans lequel au moins un parmi R¹ à R¹² est un alkyle inférieur ayant cinq carbones ou moins, ou
f) dans lequel R¹ à ¹² est sélectionné parmi le groupe constitué d'alkyles inférieurs ayant 5 carbones ou moins et l'hydrogène.

6. Procédé selon la revendication 3, dans lequel le substrat est également exposé à une source d'oxygène, et le film mince est constitué d'un oxyde de métal.

7. Procédé selon la revendication 6, dans lequel la source d'oxygène est constituée de vapeur d'eau.

8. Procédé selon la revendication 6, dans lequel la source d'oxygène est constituée de dioxygène.

9. Procédé selon la revendication 6, dans lequel la source d'oxygène est constituée d'ozone.

10. Procédé selon la revendication 3, dans lequel le substrat est également exposé à une source d'azote, et le film mince est constitué d'un nitrure de métal.

11. Procédé selon la revendication 10, dans lequel la source d'azote est constituée d'ammoniac.

12. Procédé selon la revendication 3, dans lequel
a) le film est déposé dans un processus de dépôt chimique en phase vapeur CVD, ou
b) le film est déposé dans un procédé de dépôt de couche atomique ALD, ou
c) la vapeur est obtenue en vaporisant un composé de tétra-amidinate de métal solide, ou
d) la vapeur est obtenue en vaporisant un composé de tétra-amidinate de métal liquide, ou
e) la vapeur est obtenue en vaporisant un tétra-amidinate de métal à une température située dans la plage de 100 à 250°C, ou
f) la surface est à une température située dans la plage de 200 à 500°C.
